# EUROPEAN PATENT APPLICATION

(11) **EP 3 705 132 A1**
(43) Date of publication of application: **09.09.2020**
(21) Application number: 19000111.5
(22) Date of filing: 04.03.2019
(51) Int. Cl.: A61K 38/57, A61P 27/02, G01N 33/50

(54) **METHOD FOR THE TREATMENT OF A DISEASE USING PIGMENT EPITHELIUM-DERIVED FACTOR (PEDF)**

(71) Applicant: Curebiotec GmbH, 62120 Heidelberg (DE)
(72) Inventor: Schraermeyer, Ulrich, 72379 Hechingen (DE)
(74) Representative: Bohmann, Armin K.

(57) **Abstract**

The present invention is related to a pigment epithelium-derived factor (PEDF) for use in a method for treatment and/or prevention of a disease, wherein the method comprises administering PEDF to a subject and wherein treatment and/or prevention of a disease comprises inhibiting labyrinth capillary formation, inducing growth of choriocapillaris, tightening choriocapillaris, inhibiting extracellular matrix formation, protecting choriocapillaris, and/or guiding vessel development.

## Description

The present invention is related to a pigment epithelium-derived factor (PEDF) for use in a method for treatment and/or prevention of a disease, a method for the screening of a pigment epithelium-derived factor (PEDF) analog and a method for the screening of an anti-VEGF agent

Age-related macular degeneration (AMD) is the commonest reason for legal blindness in western countries. Atrophy of the submacular retinal pigment epithelium and the development of choroidal neovascularization (CNV) results secondarily in loss of central visual acuity. Early signs of AMD are deposits (drusen) between retinal pigment epithelium and Bruch's membrane. There are two forms of AMD - wet and dry.

During the disease of wet AMD there is sprouting of choroid vessels into the subretinal space of the macula. These new vessels often develop abnormal, became leaky and cause subretinal edema. These edemas lead to loss of central vision and reading ability.

In patients with dry AMD, the primary effect is loss of the choriocapillaris (Biesemeier, Taubitz et al. 2014). Subsequently, the retinal pigment epithelium (RPE) and the photoreceptors degenerate which leads to geographic atrophy (GA). For dry AMD there is at present no treatment available to prevent loss of the choriocapillaris.

Patients with subfoveal CNV currently were treated with drugs reducing or blocking vascular endothelial growth factor (VEGF). Since 2004, anti-VEGF therapy has become the standard treatment for wet AMD and has revolutionized the management of this disease. Between 2004 and 2006, three anti-VEGF drugs were introduced to ophthalmology after receiving regulatory approval for the treatment of AMD (pegaptanib, ranibizumab) or being used off-label (bevacizumab) (Browning, Kaiser et al. 2012). They exhibit important differences in their sites of activity, formulation methods, binding affinities and biological activities (Julien, Biesemeier et al. 2014). Pegaptanib (Macugen) is an oligonucleotide aptamer that selectively binds to and neutralizes the main pathological isoform of VEGF (VEGF-A165) by attaching to its heparin-binding domain. Ranibizumab (Lucentis, Genentech/Novartis) is an affinity matured, humanized, monoclonal antibody fragment (Fab), whereas bevacizumab (Avastin, Genentech/Roche) is a full-length, humanized monoclonal antibody. Both work by blocking the receptor-binding domain of all isoforms of VEGF-A (Ferrara, Damico et al. 2006). Aflibercept (VEGF Trap-Eye, Eylea, Regeneron/Bayer) is an anti-VEGF agent recently approved by the Food and Drug Administration. It is a fully human, recombinant fusion protein composed of the second immunoglobulin (Ig)-binding domain of VEGFR1 and the third Ig-binding domain of VEGFR2 fused to the fragment crystallizable (Fc) region of human IgG1.

Aflibercept binds to all VEGF-A isoforms, VEGF-B and PlGF (Papadopoulos, Martin et al. 2012). The effects of intravitreally injected bevacizumab in the eyes of monkeys have been extensively described (Peters, Heiduschka et al. 2007, Julien, Biesemeier et al. 2013, Schraermeyer and Julien 2013). The effects included reductions in choriocapillaris fenestrations, photoreceptor damage, formation of immune complexes and thrombotic microangiopathy. A prevailing rationale for thrombosis after bevacizumab treatment was presented by Meyer and colleagues (Meyer, Robles-Carrillo et al. 2009). They found that bevacizumab can induce platelet aggregation, degranulation and thrombosis through complex formation with VEGF, heparin and activation of the platelet Fc gamma RIIa receptor. Moreover, other results have demonstrated effective binding of the Fc domain of bevacizumab to human RPE and human umbilical vascular endothelial cell membranes via Fc receptors or membrane-bound VEGF, activating the complement cascade and leading to cell death (Meyer and Holz 2011). It is unclear whether there is a similar problem with aflibercept as it also contains the Fc domain of human IgGl. Furthermore, the IgGl isotype is known to be very effective in the activation of the complement system through the classical pathway (Daha, Banda et al. 2011). Indeed, the Fc portion of IgG1 has a high ability to bind C1q causing subsequent activation of the classical pathway (Daha, Banda et al. 2011). In contrast, ranibizumab does not possess the Fc domain avoiding activation of the complement cascade, but nevertheless also induces hemolysis and fibrin formation in non-clinical studies (Julien, Biesemeier et al. 2014).

It was reported that VEGF inhibition can activate thrombocytes in humans treated for cancer (Meyer, Robles-Carrillo et al. 2009) or for neovascular AMD (Schraermeyer and Julien 2013). In addition, VEGF drugs after intravitreal application induced thrombotic microangiopathy in the choriocapillaris of monkeys (Peters, Heiduschka et al. 2007, Schraermeyer and Julien 2012). Anti-VEGF drugs also induce hemolysis, stasis and fibrin formation within the choriocapillaris (Schraermeyer and Julien 2012, Schraermeyer and Julien 2013, Julien, Biesemeier et al. 2014). Avastin forms together with heparin and VEGF protein complexes that induce thrombotic events (Julien, Biesemeier et al. 2013). In blood vessels of surgically excised choroidal membranes from patients suffering from wet AMD, anti-VEGF (bevacizumab) treatment induced thrombosis and protein complex formation (Schraermeyer, Julien et al. 2015).

These side effects are of disadvantage because AMD patients, due to their age, have higher risks to suffer from stroke or other vessel related diseases. Thus, an increased long-term mortality in individuals with wet AMD treated with bevacizumab compared to a same age and gender group without wet AMD was reported (Hanhart, Comaneshter et al. 2017). Particularly after myocardial infarction (Hanhart, Comaneshter et al. 2018) and after a cerebrovascular event (Hanhart, Comaneshter et al. 2018), the mortality caused by anti-VEGF treatment is largely enhanced. Moreover, long term treatment with anti-VEGF drugs causes loss of the original choriocapillaris and geographic atrophy in the peripheral parts of the retina in patients with wet AMD (Schutze, Wedl et al. 2015). Thus, this treatment induces additional visual loss that would not have occurred without this treatment.

Recently, due to the new possibility of using angiography together with ocular coherence tomography (OCTA) (Treister, Nesper et al. 2018) overcoming the short-coming of earlier fluorescein angiography only detecting CNV after leakage had already occurred, there is detected a notable prevalence of subclinical CNV in fellow eyes with unilateral exudative CNV, and significantly greater choriocapillaris nonperfusion adjacent to all CNV lesions. Treister et al. (Treister et al. 2018) identified a trend for increased choriocapillaris nonperfusion in exudative AMD eyes as compared with their fellow subclinical CNV eyes. This clearly shows that subclinical CNVs exist without reducing the visual acuity of these patients. These new findings support an earlier observation in eyes with late wet AMD in which neovascularization could help photoreceptors to survive (Biesemeier, Julien et al. 2014).

Regarding the long history for treatment of wet AMD, always the same principle as been used, namely removing or blocking the newly formed blood vessels. In order to achieve this, different methods have been used: laser coagulation, surgery, radiation, photodynamic therapy and today intravitreal anti-VEGF drugs. Whereas none of the first methods could improve vision, usage of anti-VEGF (ranibizumab) was successful and can improve visual acuity for a while but is still far away from an optimal rescue.

The problem underlying the present invention is the provision of a means for the treatment of ocular diseases such as age-related macular degeneration (AMD).

A further problem underlying the present invention is the provision of a means for the treatment of ocular disease such as age-related macular degeneration (AMD) providing improved visual acuity for a prolonged period of time.

These and other problems underlying the present invention are solved by the subject matter of the attached independent claims. Preferred embodiments may be taken from the attached dependent claims.

Without wishing to be bound by any theory, the present inventor has surprisingly found that pigment epithelium derived factor (PEDF) is capable of inducing growth of healthy and functional choriocapillaris and effects associated therewith such as inhibiting labyrinth capillary formation, tightening choriocapillaris and inhibiting extracellular matrix formation, which is beneficial in the treatment of eye diseases. Insofar, the present invention turns away from the current state of the art in the treatment of eye disease which is based on blocking vessel growth or removing vessels.

In light of such inhibition of labyrinth capillary formation by means of PEDF, the therapeutic effectiveness of PEDF in the treatment of eye diseases is plausible, in particular for those eye diseases showing labyrinth capillary formation such as age-related macular degeneration (AMD), both dry AMD and wet AMD, central serous chorioretinopathy, diabetic retinopathy, rubeosis iridis, corneal neovascularization, polypoidal choroidal vasculopathy and retinopathy of the prematurity. For example, if patients with wet AMD are injected with Fluorescein it is observed that high amounts of liquid leak out from the pathological vessels in a short time. The most plausible cause for this finding is that there are large gaps between or within the endothelial walls. However, gaps in the endothelium which connect blood cells and extracellular matrix would immediately be closed by thrombocytes which is not happening. Recently, capillaries with many microvilli-like projections of the endothelium which formed a labyrinth-like structure into the vessel's lumen were found in surgically excised choroidal neovascularizations (CNVs) from AMD patients. The lumen of such capillaries showed open connections towards the interstitium. These capillaries were connected to the network of blood vessels because they were filled with plasma and therefore they were a source for leakage. This type of capillary was frequently observed in CNVs and was called "labyrinth capillary". Leaky sites in these labyrinth capillaries cannot be closed by thrombocytes because due to the reduced lumen of the labyrinth capillaries thrombocytes cannot enter. Therefore, this vessel type causes chronic plasma exudation and is the origin of edema (Schraermeyer, Julien et al. 2015).

Furthermore, pigment epithelium derived factor (PEDF) has a very strong neurotrophic and neuroprotective effect (King and Suzuma 2000). This factor is produced by RPE under normoxic conditions. Production is stopped during hypoxia. This greatly promotes neovascularization. In age-related macular degeneration (AMD) the damaged RPE cells produce too little PEDF. This produces uncontrolled neoangiogenesis. It was believed that the central effect of PEDF in the eye is to prevent neogenesis of vessels (King and Suzuma 2000) but in accordance with the present invention, PEDF can stabilize CNV vessels and avoid Labyrinth capillary formation if pathological vessel formation has been initiated by VEGF.

In a first aspect, which is also a first embodiment of the first aspect, the problem underlying the present invention is solved by a pigment epithelium-derived factor (PEDF) for use in a method for treatment and/or prevention of a disease, wherein the method comprises administering PEDF to a subject and wherein treatment and/or prevention of a disease comprises inhibiting labyrinth capillary formation, inducing growth of choriocapillaris, tightening choriocapillaris, inhibiting extracellular matrix formation, protecting choriocapillaris, and/or guiding vessel development.

In a preferred embodiment thereof, PEDF is human PEDF, in a more preferred embodiment, PEDF comprises an amino acid sequence according to SEQ ID NO: 1:

QNPASPPEEG SPDPDSTGAL VEEEDPFFKV PVNKLAAAVS
NFGYDLYRVR SSTSPTTNVL LSPLSVATAL SALSLGAEQR
TESIIHRALY YDLISSPDIH GTYKELLDTV TAPQKNLKSA
SRIVFEKKLR IKSSFVAPLE KSYGTRPRVL TGNPRLDLQE
INNWVQAQMK GKLARSTKEI PDEISILLLG VAHFKGQWVT
KFDSRKTSLE DFYLDEERTV RVPMMSDPKA VLRYGLDSDL
SCKIAQLPLT GSMSIIFFLP LKVTQNLTLI EESLTSEFIH
DIDRELKTVQ AVLTVPKLKL SYEGEVTKSL QEMKLQSLFD
SPDFSKITGK PIKLTQVEHR AGFEWNEDGA GTTPSPGLQP
AHLTFPLDYH LNQPFIFVLR DTDTGALLFI GKILDPRGP (SEQ ID NO: 1)

In a preferred embodiment thereof, PEDF is a derivative of PEDF, preferably of human PEDF, and more preferably of PEDF comprising an amino acid sequence according to SEQ ID NO: 1. It will be appreciated by a person skilled in the art, that any derivative of PEDF may be used as long as the PEDF is capable of causing the above effects and in particular the effect of inhibiting labyrinth capillary formation, inducing growth of choriocapillaris, tightening choriocapillaris, inhibiting extracellular matrix formation, protecting choriocapillaris, and guiding vessel development. In an embodiment, the PEDF is one having a homology or identity to the amino acid sequence of SEQ ID NO: 1 of at least, 85%, 86%, 87 %, 88%, 89%, 90%, 91%, 92 %, 93 %, 94 %, 95 %, 96 %, 97 %, 98 % or 99 %. In a further embodiment, a derivative of PEDF is one where at amino acid position 20 of SEQ ID NO: 1 the amino acid residue is pyrrolidone carboxylic acid, at amino acid position 24 of SEQ ID NO: 1 the amino acid residue is phosphoserine, at amino acid position 114 of SEQ ID NO: 1 the amino acid residue is phosphoserine, at amino acid position 227 of SEQ ID NO: 1 the amino acid residue is phosphoserine and/or at amino acid position 285 of SEQ ID NO: 1 the amino acid residue is N-linked (GlcNAc) asparagine.

It will be appreciated that any of the above effects and, in particular, labyrinth capillary formation and any change thereof including inhibition thereof, induction of growth of choriocapillaris, tightening of choriocapillaris, inhibition of extracellular matrix formation, protection of choriocapillaris, and guidance of vessel development can be assessed by optical coherence tomography (OCT-A), preferable when combined with fluorescein angiography (FA) which is suitable for detecting and assessing, respectively, leaking vessels (Spaide et al. 2015). Optical coherence tomography angiography (OCT-A) emerged as a non-invasive technique for imaging the microvasculature of the retina and choroid (Spaide et al.2015). Briefly, OCT-A technology uses laser light reflectance of the surface of moving red blood cells to accurately depict vessels through different segmented areas of the eye, thus eliminating the need for intravascular dyes. The OCT scan of a patient's retina consists in multiple individual A-scans, which compiled into a B-scan provides cross-sectional structural information. With OCT-A technology, the same tissue area is repeatedly imaged and differences analyzed between scans, thus allowing one to detect zones containing high flow rates, i.e. with marked changes between scans, and zones with slower, or no flow at all, which will be similar among scans.

OCT-A and FA may also be used for the detection and assessment, respectively, of edema which are located within the retina and/or subretinal space.

In a second embodiment of the first aspect, which is also an embodiment of the first embodiment of the first aspect, the disease is an eye or ocular disease.

In a third embodiment of the first aspect, which is also an embodiment of the first and second embodiment of the first aspect, the eye disease is macular degeneration, preferably age-related macular degeneration (AMD), more preferably dry age-related macular degeneration of wet age-related macular degeneration.

In a fourth embodiment of the first aspect, which is also an embodiment of the first and second embodiment of the first aspect, the eye disease is selected from the group comprising central serous chorioretinopathy, diabetic retinopathy, rubeosis iridis, corneal neovascularization, polypoidal choroidal vasculopathy and retinopathy of the prematurity.

In a fifth embodiment of the first aspect, which is also an embodiment of the first, second, third and fourth embodiment of the first aspect, labyrinth capillary formation is labyrinth capillary formation in an eye, preferably in eye disease.

In a sixth embodiment of the first aspect, which is also an embodiment of the first, second, third, fourth and fifth embodiment of the first aspect, inducing growth of choriocapillaris comprises or is inducing growth of new choriocapillaris.

In a seventh embodiment of the first aspect, which is also an embodiment of the first, second, third, fourth, fifth and sixth embodiment of the first aspect, inducing growth of choriocapillaris provides choriocapillaris which are capable of replacing original choriocapillaris, preferably original choriocapillaris are diseased choriocapillaris. In connection therewith, it will be acknowledged by a person skilled in the art that diseased choriocapillaris is located between Bruch's membrane and RPE and can be seen in OCT-A.

In an eight embodiment of the first aspect, which is also an embodiment of the first, second, third, fourth, fifth, sixth and seventh embodiment of the first aspect, wherein tightening choriocapillaris comprises tightening pathological choriocapillaris. In connection therewith, it will be acknowledged that each neovascular choriocapillaris or vessel located between Bruch's membrane and RPE or within the subretinal space is preferably regarded as pathologic. More preferably, a choriocapillaris is regarded as pathologic only when they develop into labyrinthy capillaries or became leaky by other reasons. Diagnosis thereof may be performed by OCT-A and/or fluorescein angiography (FA).

In a ninth embodiment of the first aspect, which is also an embodiment of the first, second, third, fourth, fifth, sixth, seventh and eighth embodiment of the first aspect, inhibiting extracellular matrix formation comprises inhibition of extracellular matrix formation towards the lumen of a blood vessel and/or around a blood vessel. Preferably, such vessel does not inhibit the flow of red blood cells by absence of endothelial projection into the lumen.

In a tenth embodiment of the first aspect, which is also an embodiment of the first, second, third, fourth, fifth, sixth, seventh, eighth and ninth embodiment of the first aspect, protecting choriocapillaris comprises protecting choriocapillaris from the damaging effect of an anti-VEGF drug. Such anti-VEGF drug is preferably one selected from the group comprising pegaptanib, ranibizumab, bevacizumab and aflibercept. In connection therewith, it will be acknowledged that such damaging effect may encompass regression of blood vessels and degeneration of RPE and photo receptors resulting in geographic atrophy. Geographic atrophy may be detected as a dark spot upon scanning laser ophthalmoscopy (SLO) because autofluorescence of the RPE disappears. The SLO picture is the result of autofluorescence of lipofuscin in RPE.

In an eleventh embodiment of the first aspect, which is also an embodiment of the first, second, third, fourth, fifth, sixth, seventh, eighth, ninth and tenth embodiment of the first aspect, protecting choriocapillaris comprises protecting choriocapillaris from the damaging effect of withdrawal of an anti-VEGF drug. In connection therewith, it will be appreciated that, preferably, blood vessels become leaky and change into labyrinth capillaries; endothelial cells proliferate and migrate.

In a twelfth embodiment of the first aspect, which is also an embodiment of the first, second, third, fourth, fifth, sixth, seventh, eighth, ninth, tenth and eleventh embodiment of the first aspect, guiding vessel development comprises development of a functional blood vessel, preferably a functional blood vessel from a pathological blood vessel. In connection therewith, preferably a pathological blood vessel is a blood vessel which does not allow proper blood flow, is leaky of forms too many or atypical extracellular matrix proteins.

In a 13^{th} embodiment of the first aspect, which is also an embodiment of the twelfth embodiment of the first aspect, the pathological blood vessel is the result of a pathological condition. Such pathological condition may be one or a combination of hypoxia, upregulation of HIF 1 alpha, atypical formation of growth factors and VEGF.

In a 14^{th} embodiment of the first aspect, which is also an embodiment of the first, second, third, fourth, fifth, sixth, seventh, eighth, ninth, tenth, eleventh, twelfth and 13^{th} embodiment of the first aspect, PEDF is administered intravitreally or sub-retinally, or as a vector such as adeno-associated virus coding for PEDF.

In a 15^{th} embodiment of the first aspect, which is also an embodiment of the first, second, third, fourth, fifth, sixth, seventh, eighth, ninth, tenth, eleventh, twelfth, 13^{th} and 14^{th} embodiment of the first aspect, the method further comprises applying an anti-VEGF therapy, preferably the anti-VEGF therapy comprises administration to the subject of an anti-VEGF drug, wherein the anti-VEGF drug is selected from the group comprising pegaptanib, ranibizumab, bevacizumab and aflibercept. In connection therewith, it will be acknowleged ba by a person skilled in the art that PEDF may be used early, for example when the CNV is detected in one eye, the fellow eye may be treated prophylactically; also if a subclinical CNV with normal vision of the eye is diagnosed the treatment may begin in order to keep the CNV stable.

In a 16^{th} embodiment of the first aspect, which is also an embodiment of the first, second, third, fourth, fifth, sixth, seventh, eighth, ninth, tenth, eleventh, twelfth, 13^{th}, 14^{th} and 15^{th} embodiment of the first aspect, the subject is subject who is suffering from side effects of anti-VEGF treatment, preferably visual loss arising from anti-VEGF treatment.

In a second aspect, which is also a first embodiment of the second aspect, the problem underlying the present invention is solved by an mRNA coding for a pigment epithelium-derived factor (PEDF) for use in a method for treatment and/or prevention of a disease, wherein the method comprises administering PEDF to a subject and wherein treatment and/or prevention of a disease comprises inhibiting labyrinth capillary formation, inducing growth of choriocapillaris, tightening choriocapillaris, inhibiting extracellular matrix formation, protecting choriocapillaris, and/or guiding vessel development. In an embodiment, the mRNA is an mRNA coding for the amino acid sequence according to SEQ ID NO: 1. In an alternative embodiment, the mRNA is a nucleotide sequence of SEQ ID NO: 2:

GGACGCTGGATTAGAAGGCAGCAAAAAAAGATCTGTGCTGGCTGGAGCCCCCTC
AGTGTGCAGGCTTAGAGGGACTAGGCTGGGTGTGGAGCTGCAGCGTATCCACAG
GCCCCAGGATGCAGGCCCTGGTGCTACTCCTCTGCATTGGAGCCCTCCTCGGGCA
CAGCAGCTGCCAGAACCCTGCCAGCCCCCGGAGGAGGGCTCCCCAGACCCCGA
CAGCACAGGGGCGCTGGTGGAGGAGGAGGATCCTTTCTTCAAAGTCCCCGTGAA
CAAGCTGGCAGCGGCTGTCTCCAACTTCGGCTATGACCTGTACCGGGTGCGATCC
AGCATGAGCCCCACGACCAACGTGCTCCTGTCTCCTCTCAGTGTGGCCACGGCCC
TCTCGGCCCTCTCGCTGGGAGCGGACGAGCGAACAGAATCCATCATTCACCGGGC
TCTCTACTATGACTTGATCAGCAGCCCAGACATCCATGGTACCTATAAGGAGCTC
CTTGACACGGTCACTGCCCCCCAGAAGAACCTCAAGAGTGCCTCCCGGATCGTCT
TTGAGAAGAAGCTRCGCATAAAATCCAGCTTTGTGGCACCTCTGGAAAAGTCATA
TGGGACCAGGCCCAGAGTCCTGACGGGCAACCCTCGCTTGGACCTGCAAGAGAT
CAACAACTGGGTGCAGGCGCAGATGAAAGGGAAGCTCGCCAGGTCCACAAAGGA
AATTCCCGATGAGATCAGCATTCTCCTTCTCGGTGTGGCGCACTTCAAGGGGCAG
TGGGTAACAAAGTTTGACTCCAGAAAGACTTCCCTCGAGGATTTCTACTTGGATG
AAGAGAGGACCGTGAGGGTCCCCATGATGTCGGACCCTAAGGCTGTTTTACGCTA
TGGCTTGGATTCAGATCTCAGCTGCAAGATTGCCCAGCTGCCCTTGACCGGAAGC

ATGAGTATCATCTTCTTCCTGCCCCTGAAAGTGACCCAGAATTTGACCTTGATAG
AGGAGAGCCTCACCTC (SEQ ID NO: 2)

In a further embodiment of the second aspect, including any embodiment thereof, the mRNA is a recombinant or heterologous mRNA which preferably comprises a structural element such as a 5' UTR and/or 3' UTR, which is different from the 5' UTR and/or 3' UTR of the mRNA form which the coding sequence of PEDF is taken.

The disclosure of the first aspect, including any embodiment thereof, equally apply to the second aspect. In other words, any embodiment of the first aspect is also an embodiment of the second aspect.

In a third aspect, which is also a first embodiment of the third aspect, the problem underlying the present invention is solved by a pharmaceutical composition either comprising a pigment epithelium-derived factor (PEDF) or an mRNA coding for a pigment epithelium-derived factor (PEDF), wherein the pharmaceutical composition is for use in a method for treatment and/or prevention of a disease, wherein the method comprises administering PEDF to a subject and wherein treatment and/or prevention of a disease comprises inhibiting labyrinth capillary formation, inducing growth of choriocapillaris, tightening choriocapillaris, inhibiting extracellular matrix formation, protecting choriocapillaris, and/or guiding vessel development. Preferably, the pharmaceutical composition comprises a pharmaceutically acceptable excipient or diluent.

The disclosure of the first aspect and the second aspect, including any embodiment thereof, equally apply to the third aspect. In other words, any embodiment of the first aspect and the second aspect is also an embodiment of the third aspect.

In a fourth aspect, which is also a first embodiment of the fourth aspect, the problem underlying the present invention is solved by the use of a pigment epithelium-derived factor (PEDF) or an mRNA coding for a pigment epithelium-derived factor (PEDF) for the manufacture of a medicament for the treatment and/or prevention of a diseases, wherein treatment and/or prevention of a disease comprises inhibiting labyrinth capillary formation, inducing growth of choriocapillaris, tightening choriocapillaris, inhibiting extracellular matrix formation, protecting choriocapillaris, and/or guiding vessel development.

The disclosure of the first aspect and the second aspect, including any embodiment thereof, equally apply to the fourth aspect. In other words, any embodiment of the first aspect and the second aspect is also an embodiment of the fourth aspect.

In a fifth aspect, which is also a first embodiment of the fifth aspect, the problem underlying the present invention is solved by a method for the treatment and/or prevention of a disease in a subject, wherein treatment and/or prevention of a disease comprises administering to the subject a therapeutically effective amount of a pigment epithelium-derived factor (PEDF) or an mRNA coding for a pigment epithelium-derived factor (PEDF) and inhibiting labyrinth capillary formation, inducing growth of choriocapillaris, tightening choriocapillaris, inhibiting extracellular matrix formation, protecting choriocapillaris, and/or guiding vessel development.

The disclosure of the first aspect and second aspect, including any embodiment thereof, equally apply to the fifth aspect. In other words, any embodiment of the first aspect and the second is also an embodiment of the fifth aspect.

In a sixth aspect, which is also a first embodiment of the sixth aspect, the problem underlying the present invention is solved by a pigment epithelium-derived factor (PEDF) for use, in a subject, in a method for inhibiting labyrinth capillary formation, inducing growth of choriocapillaris, tightening choriocapillaris, inhibiting extracellular matrix formation, protecting choriocapillaris, and/or guiding vessel development, and wherein the method comprises administering PEDF to the subject.

The disclosure of the first aspect, including any embodiment thereof, equally apply to the sixth aspect. In other words, any embodiment of the first aspect is also an embodiment of the sixth aspect.

In a seventh aspect, which is also a first embodiment of the seventh aspect, the problem underlying the present invention is solved by an mRNA coding for a pigment epithelium-derived factor (PEDF) pigment epithelium-derived factor (PEDF) for use, in a subject, in a method for inhibiting labyrinth capillary formation, inducing growth of choriocapillaris, tightening choriocapillaris, inhibiting extracellular matrix formation, protecting choriocapillaris, and/or guiding vessel development, and wherein the method comprises administering PEDF to the subject.

The disclosure of the first aspect and second aspect, including any embodiment thereof, equally applies to the seventh aspect. In other words, any embodiment of the first and second aspect is also an embodiment of the seventh aspect.

In an eighth aspect, which is also a first embodiment of the eighth aspect, the problem underlying the present invention is solved by a method for the screening of a pigment epithelium-derived factor (PEDF) analog, wherein the method comprises
- intravitreally or subretinally administering VEGF into an animal model,
- administering a pigment epithelium-derived factor (PEDF) analog candidate into the animal model,
- determining the effect of the pigment epithelium-derived factor (PEDF) analog candidate after 1 to 72 h,
wherein the pigment epithelium-derived factor (PEDF) analog candidate is a pigment epithelium-derived factor (PEDF) analog if the effect of VEGF is blocked, no leakage of the blood vessels occurs, no increase in the extracellular matrix occurs and/or no thickening of the Bruch's membrane occurs.

In a ninth aspect, which is also a first embodiment of the ninth aspect, the problem underlying the present invention is solved by a method for the screening of an anti-VEGF agent, wherein the method comprises
- intravitreally or subretinally administering VEGF into an animal model,
- administering an anti-VEGF agent candidate into the animal model
- determining the effect of the anti-VEGF agent candidate after 1 to 72 h,
wherein the anti-VEGF agent candidate is an anti-VEGF agent if the effect of VEGF is blocked, no leakage of the blood vessels occurs, no increase in the extracellular matrix occurs, and/or no thickening of the Bruch's membrane occurs.

In a second embodiment of the eighth and the ninth aspect, which is also an embodiment of the first embodiment of the eighth and ninth aspect, the animal model is the vitreous or subretinal space of an animal, preferably the animal is selected from the group comprising a mouse, a rat, a guinea pig, a pig, a monkey and an ape.

In a third embodiment of the eighth and the ninth aspect, which is also an embodiment of the first and second embodiment of the eighth and ninth aspect, VEGF and the PEDF analog candidate of the anti-VEGF agent candidate may be administered sequentially or together.

In a fourth embodiment of the eighth and the ninth aspect, which is also an embodiment of the first, second and third embodiment of the eighth and ninth aspect, the effect of the pigment epithelium-derived factor (PEDF) analog candidate and, respectively, the anti-VEGF agent candidate on the effect of VEGF on blood vessels, preferably blood vessels in the eye, more preferably choriocapillaris, the effect on leakage of such blood vessels, the effect on increase in extracellular matrix and/or the effect on thickening of the Bruch's membrane is determined.

In a fifth embodiment of the eighth and the ninth aspect, which is also an embodiment of the fourth embodiment of the eighth and ninth aspect, the effect is one arising from the VEGF applied to the animal model.

In a sixth embodiment of the eighth and the ninth aspect, which is also an embodiment of the first, second, third, fourth and fifth embodiment of the eighth and ninth aspect, the effect is determined by a means selected from the group comprising electron microscopy, cytochemistry and molecular biology.

In a seventh embodiment of the eight and the ninth aspect which is also an embodiment of the sixth embodiment of the eighth and ninth aspect, the means selected from molecular biology comprises reverse PCR (RT-PCR) and characterization of proteins by mass spectrometry.

In an eighth aspect of the eighth and the ninth aspect, which is also an embodiment of the first, second, third, fourth, fifth, sixth and seventh embodiment of the eighth and ninth aspect, VEGF is human VEGF.

In connection with the screening method according to the eighth and ninth aspect, it will be appreciated by a person skilled in the art that if the PEDF analog candidate and the anti-VEGF agent candidate, respectively, is administered sub-retinally, the above effects may be observed as early as one hour after administration. In connection with the screening method according to the eighth and ninth aspect, it will also be appreciated by a person skilled in the art that if the PEDF analog candidate and the anti-VEGF agent candidate, respectively, is administered intravitreally, the above effects may be observed as early as 12 to 24 hours after administration.

The present invention is further illustrated by the figures, examples and sequence listing from which further features, embodiment and advantages may be taken. In connection therewith,
Fig. 1 is an electron micrograph showing a choriocapillaris of an untreated rat that was fixed directly after enucleation; the arrows mark the fenestrations in the endothelium towards Bruch's membrane; the indicated bar = 2 µm;
Fig. 2 is an electron micrograph taken fourteen hours after hypoxia; there were many filopodia-like projections within the capillary lumen which is largely reduced; the extracellular matrix surrounding the capillary was enhanced (arrowhead) and cells appeared within Bruch's membrane (arrow); the indicated bar = 2 µm;
Fig. 3 is an electron micrograph taken after hypoxia; individual filipodia of the endothelium projected into the capillary lumen were more than 10 µm long (arrows); the indicated bar = 2 µm
Fig. 4 is an electron micrograph taken after hypoxia; were many open gaps between or within the endothelial cells (arrowhead) of the labyrinth capillaries; the indicated bar = 2 µm;
Fig. 5 is an electron micrograph taken after intravitreal PEDF injection and hypoxia; labyrinth capillaries did not develop and the lumen of the capillaries was maintained like in vivo (asterisk); the indicated bar = 5 µm;
Fig. 6 is an electron micrograph taken after hypoxia and without intravitreal PEDF injection; labyrinth capillaries developed and the lumen of the capillaries was collapsed (arrow); the indicated bar = 5 µm;
Fig. 7 is a bar diagram showing quantitative analysis of the areas occupied by the choricapillaris, by the choriocapillaris lumen and by the endothelium in ultrathin section after hypoxia and treatment with Avastin, PEDF or without treatment;
Fig. 8 is an electron micrograph of a CNV shown in a semithin section; the left and right arrow mark the extension of the CNV and the site where the RPE remains a monolayer. The photoreceptor nuclear layer is thinner and the outer segments are irregular facing the CNV;
Fig. 9 shows a representative SLO angiography image about 20 min after injection of dyes (left fluorescein angiography (FA), right (indocyanine green angiography (ICG)) for an eye six weeks after VEGF-vector injection;
Fig. 10 is a bar diagram showing the means of change of the maximal thickness of the CNV lesion area between the measurements six (pretreatment) and seven weeks after vector injection (one week after treatment) for each group; Mean standard deviations are shown * = p < 0.05, *** = p < 0.0001;
Fig. 11 is an electron micrograph showing a newly formed choriocapillaris located between Bruch's membrane (black arrowhead) and RPE; the vessel contains a red blood cell (RB); between RPE and the new vessel a new Bruch's membrane (white arrowhead) was been formed after PEDF treatment;
Fig 12 is an electron micrograph showing a newly formed choriocapillaris located between Bruch's membrane (black arrowhead) and RPE; the vessel contains a red blood cell (RB); between RPE and the new vessel a new Bruch's membrane (white arrowhead) was been formed; a pericyte (P) is associated to this vessel which also is fenestrated (arrows) in the endothelium facing the RPE after PEDF treatment;

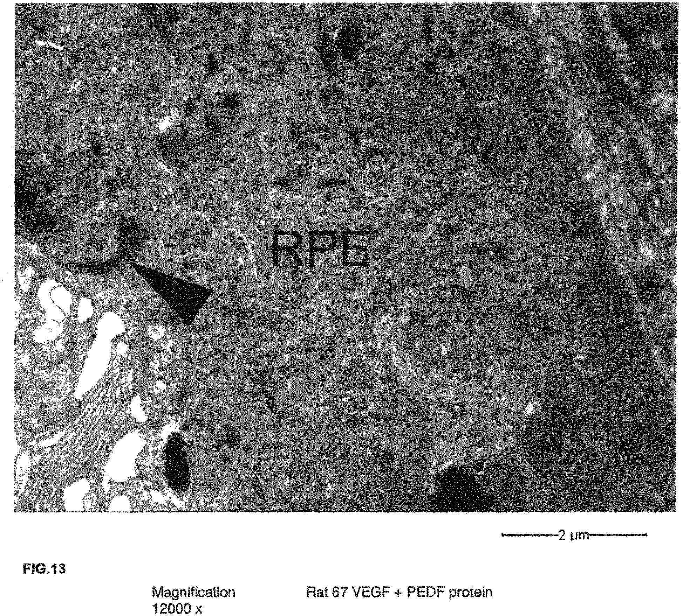

Fig. 13 is an electron micrograph showing extremely electron-dense tight junctions (arrowhead) between two RPE cells after PEDF treatment;
Fig 14 is an electron micrograph showing several extremely electron dense and prominent junctions (arrowheads) between two endothelial of a choriocapillaris cells after PEDF treatment; and

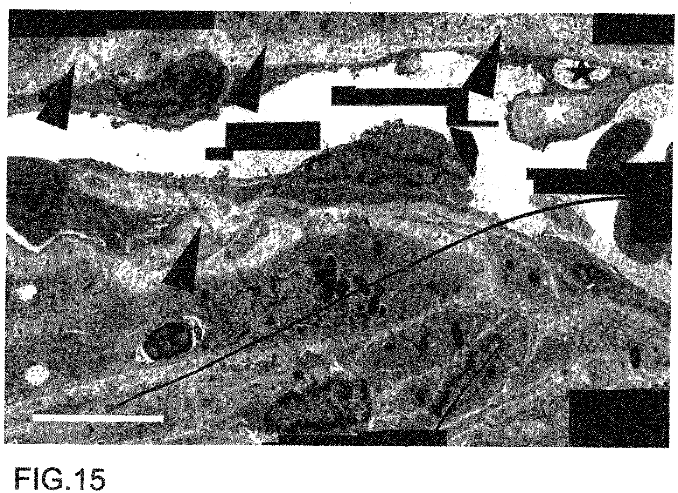

Fig. 15 is an electron micrograph shows a newly formed blood vessels which are surrounded by a thick layer of extracellular matrix (arrowheads) and separated by several layer of RPE cells from the original RPE monolayer in the absence of PEDF treatment; a protusion of extracellular matrix shifted an endothelium fold towards the vessel lumen (white asterisk); within the endothelium a large vacuole is formed (black asterisk) which is typical for pathological vessels in human CNV (Schraermeyer, Julien et al. 2015); such protrusions or vacuoles were not seen after PEDF treatment; the indicated bar = 5 µm.

### Example 1: Exposure of eyes to hypoxia

Thirty-two eyes from 16 rats were exposed to mild hypoxia. Ischemia was modeled by incubating the eyes in DMEM at 4°C during fourteen hours after enucleation in 15 ml Falcon tubes (1 eye per tube). Hypothermia can prolong the tolerance time to an ischemic insult. The tubes were filled with 7 ml DMEM and air. They were stored horizontally to enhance the oxygen exchange between DMEM and air. After 14 hours half of the eyes were embedded into paraffin for immunocytochemistry or into Epon for electron microscopy. Twelve eyes were embedded directly after enucleation and served as controls.

The oxygen pressure was measured by a calibrated fiber optic oxygen sensor (WPI, Friedberg, Germany) which was inserted into the vitreous body of eyes in this ex vivo experiment and for comparison in eyes of living rats under anesthesia. Directly after enucleation the oxygen pressure dropped down to 2% of the in vivo concentration and then gradually increased and reached the in vivo concentration after 1 hour. After that the in vivo oxygen concentration was not undercut.

### Example 2: Measurement of the inner circumferential contour of the filipodia-like projections of the endothelial cells

Electron micrographs from choriocapillaris vessels from each plastic embedded eye were analyzed for the inner circumferential contour of the filopodia-like projections. Also, the length of the outer endothelial cell circumferential contour per sectioned vessel area was measured. The iTEM image analysis software (iTEM version 5.0; Olympus Soft Imaging Solutions, Münster, Germany) was used for the measurements. The results were analyzed in Microsoft Excel 2011 and IBM SPSS Statistics 22 software by using a nonparametric Mann-Whitney test. A p-value of less than 0.05 was considered significantly different between groups. The length of the inner endothelial cell circumferential contour increased by 58% (p<0.001) in comparison to the control group which indicates the formation of microvillar endothelial cell projections towards the vessel lumen. These vessels correspond exactly to the labyrinth capillaries in human CNV (Schraermeyer, Julien et al. 2015).

### Example 3: Effect of hypoxia on choriocapillaris

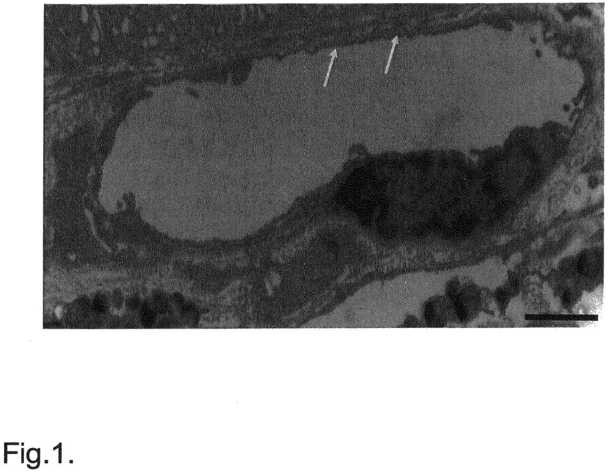

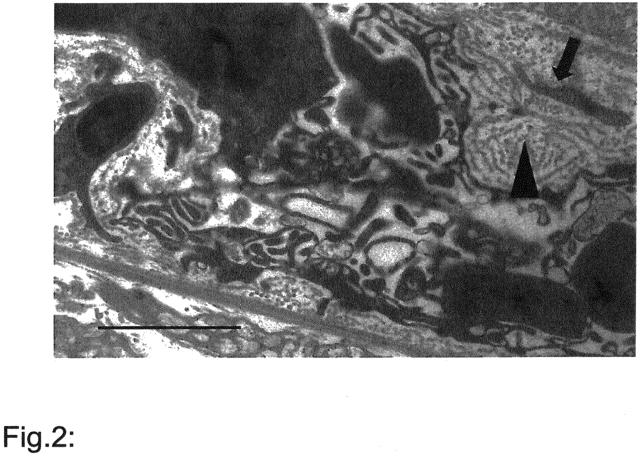

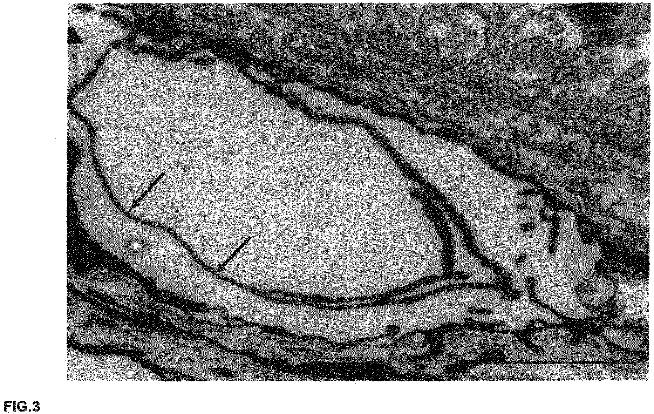

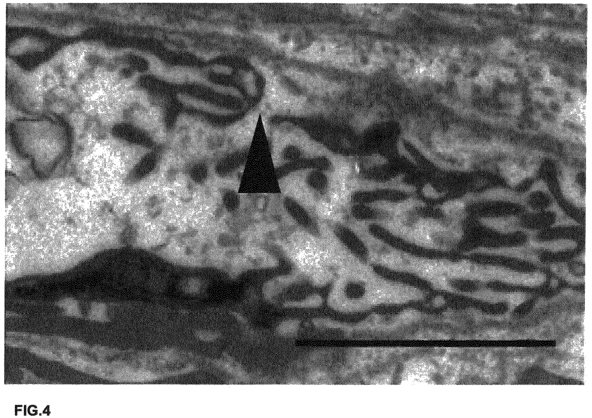

The choriocapillaris without exposure to hypoxia contains a regular thin endothelium with fenetrations towards the side of Bruch's membrane (see, Fig.1 arrows). The lumen of the capillaries is lacking any cellular projections. Fourteen hours after hypoxia there were many filopodia-like projections within the capillary lumen. (see, Fig.2). The extracellular matrix surrounding the capillary was enhanced (arrowhead) and cells appeared within Bruch's membrane (arrow). Individual filipodia within the capillary lumen were more than 10 µm long (see, Fig.3 arrows). After hypoxia there were many open gaps between or within the endothelial cells (see, Fig.4 arrowhead).

### Example 4: Expression of VEGF and HIF-1α after Hypoxia

Control and ischemic eyes were formalin-fixed and paraffin-embedded according to standard procedures. 4-µm thick sections were cut, deparaffinized and rehydrated, and boiled in a citrate buffer (pH=6.0). After three washing steps in TBS (pH=7.6), immuno-histochemical staining of HIF-1α and VEGF were performed according to the instructions provided by the manufacturer in a humid chamber. The slides were incubated for 120 min with the primary rabbit anti-HIF-1α antibody (1:100, Abcam, Denmark) at 37°C and then processed using the DAKO REAL Detection System Alkaline Phosphatase/RED kit rabbit/mouse, then counterstained with hematoxylin and covered. The same procedure was performed for immune reactivity analysis against VEGF using a primary mouse antibody (1:50, Gene Tex, USA) and boiling in TBS buffer (pH=9.0).

In control rats, HIF-1α was not expressed in the choroid. After Hypoxia HIF-1α was detected in the choroid. VEGF in the control eyes was detected within the RPE. After 14 hours of ischemia the staining of VEGF appeared additionally in the retina and the choroid.

### Example 5: Inhibition of labyrinth capillary formation by PEDF

12 eyes were injected with 20 µg PEDF (BioVendor) and then exposed to hypoxia as described in example 1. Three eyes were injected with 0.8 µl Bevacizumab (Avastin). Six eyes were also exposed to hypoxia without any injection. Ultrathin sections of the eyes were investigated under the electron microscope.

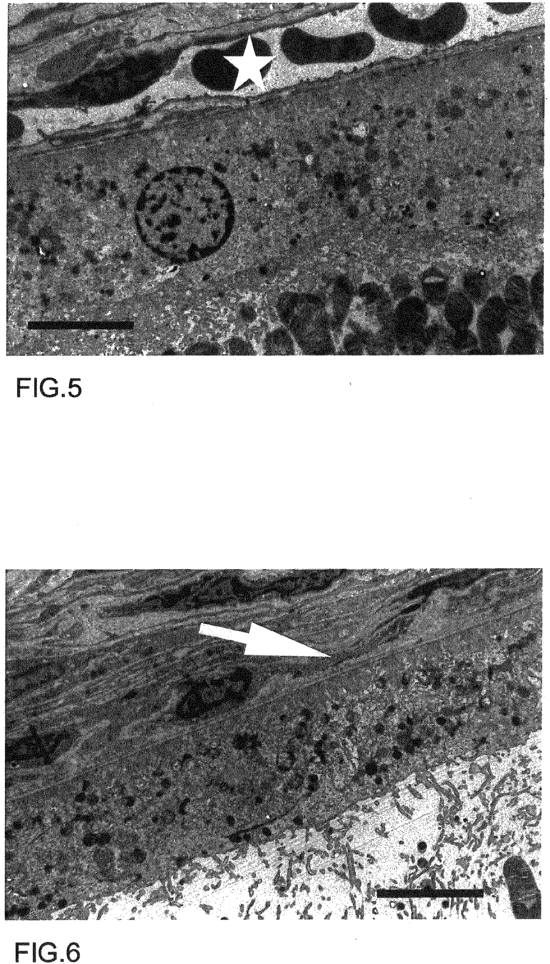

Without treatment the choriocapillaris changed into labyrinth capillaries with gaps between the endothelium as shown in Figs. 1 to 4 and collapsed leading to often complete loss of the capillary lumen (see, arrow in Fig.5). In contrast the lumen of the choriocapillaris appeared like after in-vivo fixation and were well preserved (see, asterisk in Fig.6).

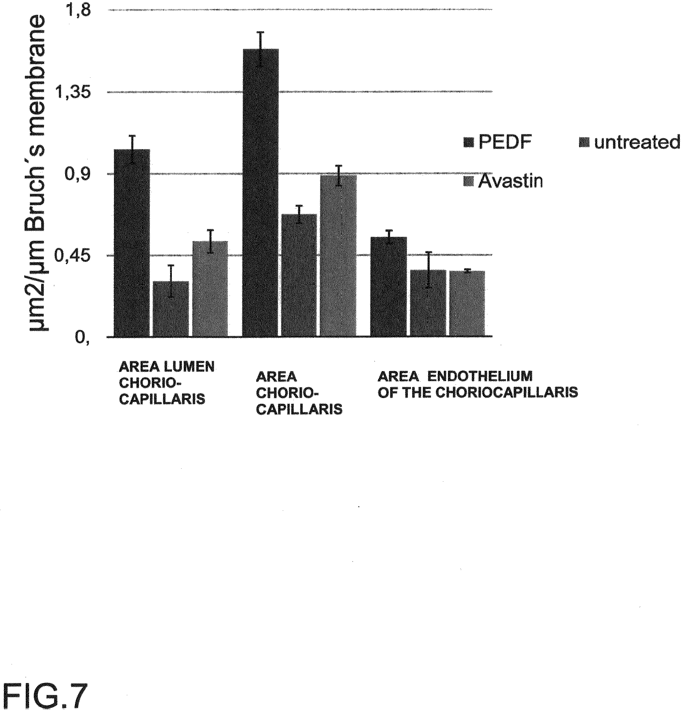

The areas enclosed by the inner and outer endothelial cell circumferential contour per sectioned vessel were measured in electron micrographs from all eyes. From these measurements the areas occupied by the entire choriocapillaris, by the lumen of the choriocapillaris and by the endothelium were calculated. PEDF not only inhibited the formation of endothelial filopodia towards the vessel lumen and gaps, it also preserved the vessel lumina significantly better than without treatment (see, Fig.7) (p< 0.0000003) and compared to Avastin treatment (p < 0.023). Also, the area of the sectioned endothelial cells, which is likely proportional to the volume of the cells, was significantly larger compared to untreated (see, Fig.7 right) (p < 0.03) whereas Avastin had no effect (p = 0.75).

Students t-test was performed to compare the results of the different experimental groups. For the analysis the excel software was used. The error probability was 5% (p < 0.05 statistically significant).

### Example 6: Formation of functional tight choriocapillaris and Bruch's membrane after VEGF overexpression and PEDF treatment

A new vector system was designed for this project, using the same VEGF cassette as in the adeno-vector studies before (Julien, Kreppel et al. 2008). Human VEGF-A165 cDNA, from the plasmid pBLAST49- hVEGF (Invivogen, San Diego, CA) was inserted in a state of the art AAV2 vector (subtype 4) backbone produced by Sirion Biotech GmbH (Munich, Germany). The new AAV vector has the benefit that it contains an RPE specific RPE65 promotor instead of the unspecific CMV promotor used before in adenoviral studies. These new AAV-vectors (e.g. AAV-VEGF) are less toxic and have a slower expression rate with longer expression time as compared to the adeno-vectors, favorable for long time expression studies dedicated for evaluation of drug candidates for treatment over a time frame of several months (Rolling, Le Meur et al. 2006).

### Subretinal injection of AAV.VEGF-A165 vector in rat eyes

2x109 virus particles of the AAV-VEGF vector, diluted in 2 µl PBS were sub-retinally injected in both eyes of 30 Long Evans rats. Briefly, after anaesthesia with an intraperitoneal injection of a three component narcosis (0.005 mg fentanyl, 2 mg midazolam and 0.15 mg of medetomidine/kg body weight), the pupils were dilated with 1 to 2 drops of Medriaticum drops (Pharmacy of the University of Tübingen, Germany) and a drop of topical anaesthetic Novesine (OmniVision, Puchheim, Germany) was applied. Methocel (OmniVision, Puchheim, Germany) eye drops were used to avoid drying of the eyes. Injections were performed using a surgical microscope. The sclera was first opened with a 25 G needle close to the limbus, then 2 µl of vector suspension (2 µl contain 2x109 virus particles AAV-VEGF, max. possible dose) were injected sub-retinally (pars plana) using a 10 µl NanoFil syringe with a NanoFil 34 G blunt needle (World Precision Instruments). Topical antibiotic eye drops Gentamicin-POS® (Ursapharm, Saarbrücken, Germany) were applied after the injection. The anaesthesia was neutralized by subcutaneous injection of an antidote (0.12 mg naloxon, 0.2 mg flumazenil, 0.75 mg atipamezol/kg body weight).

### Intravitreal injection

### Intravitreal injection of the therapeutic substances was made 6 weeks after VEGF vector injection

For the intravitreal injections, a small incision was made into the conjunctiva at the outer corner of the eyes. The eyeball was rotated by grasping the conjunctiva with a pair of fine tweezers and gentle pulling. A volume of 5 µl was injected through the hole intravitreally using a 10 µl NanoFil syringe with a NanoFil 34 gauge bevelled needle (World Precision Instruments). After the injection, the needle remained in the eye for an additional 3 or 4 seconds to reduce reflux and was then drawn back. The eyeball was brought back into its normal position, and the antibiotic ointment was applied to the eye. The whole procedure was performed using a surgical microscope equipped with illumination. Three groups were investigated.
1) Avastin® (bevacizumab; 25 mg/ml; Roche) was injected intravitreally into 20 eyes: It was purchased and aliquoted by the Pharmacy of the University Hospital of Tübingen. 100 mg of Avastin® were diluted in four milliliters of the vehicle solution contains 240 mg a,a-trehalose 2 H2O, 23.2 mg Na2HPO4 H2O, 4.8 mg NaH2PO4, and 1.6 mg polysorbate 20.
2) PEDF human HEK293 recombinant protein (1 µg/µl; BioVendor) was injected intravitreally into 20 eyes.

The pellet of the of the recombinant protein was filtered (0.4 µm) and lyophilized in 0.5 mg/mL in 20mM TRIS, 50mM NaCl, pH 7.5. According to the product data sheet, it was dissolved in deionized water (Ampuwa water) in order to obtain a working stock solution of 1 µg/µl.
3) 20 eyes were not treated
In vivo imaging (SLO/OCT, FA and ICG angiographies) and quantifications were performed according to (Wang, Rendahl et al. 2003). Subretinal AAV-VEGF leads to RPE proliferation 5 weeks to 20 months after injections, leakage can be observed starting from 2-12 months by fluorescein angiography. Therefore, scanning laser ophthalmoscopy (SLO), optical coherence tomography (OCT), fluorescein angiography (FA) and indocyanine green angiography (ICG) were performed 6 weeks after vector injection. The eyes were reinvestigated 7 weeks after injection of the VEGF vector using a SpectralisTM HRA+OCT (Heidelberg Engineering, Heidelberg, Germany) device modified for the use with animals according to protocols from (Fischer, Huber et al. 2009, Huber, Beck et al. 2009). A 78 dpt double aspheric lens (Volk Optical, Inc., Mentor, OH 44060, U.S.A.) was placed directly to the outlet of the device, an additional custom-made +3.5 dpt contact lens directly on the eyes of the rats. The rats were anaesthetized, the pupils dilated and treated with Methocel to avoid drying of the eyes and for better adherence of the 3.5 dpt lens. The ICG dye (250 µl (VERDYE, 5 mg/ml, Diagnostic Green) was injected into the tail vein, the fluorescein dye (Alcon 10% (1/10 dilution), 250 µl) was injected subcutaneously. SLO/OCT was performed ca. 2 to 5 minutes after injection for early phase and ca 15 to 20 minutes later for late phase angiography imaging. As the SLO/OCT machine is calibrated for the use with human eyes, the dimension in the x and y axis are not corrected for use in rats. Dimensions in the z axis, like retinal height, are displayed properly. Therefore, measurements of CNV hyper-fluorescent areas in the angiography measurements performed here are presented in arbitrary units (au) and not in µm using the original Heidelberg calibration. Quantification of the thickness measurements performed in OCT data sets is displayed in µm as they lay in the z direction of the beam.

### Processing of the eyes for histology

For electron microscopy (EM), whole eyes were fixed in 5 % glutaraldehyde in 0.1 M cacodylate buffer (pH 7.4) over night. Then, it will be possible to cut the lesion area, as it was visible in the angiography, and to embed it.

### Statistics

Students t-test was performed to compare the results of the treated animals with the control groups. For the analysis the excel software was used. The error probability was 5% (p < 0.05 statistically significant). To avoid the multiple comparisons problem, the results were corrected using the Holm-Bonferroni method.

### Investigation of CNV 6 weeks after subretinal injection of AAV.VEGF-A165 in rat eyes by angiography

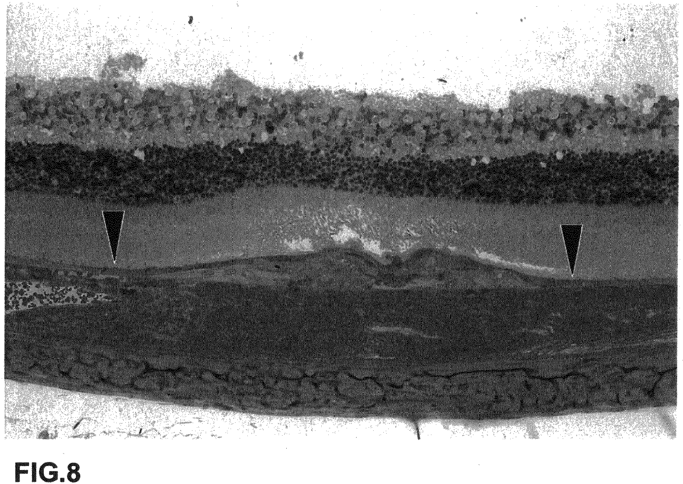

The AAV-VEGF triggered rat CNV model showed a fully grown CNV 6 weeks after VEGF transduction, as documented by in vivo imaging. A representative image is presented in (see, Fig. 8).

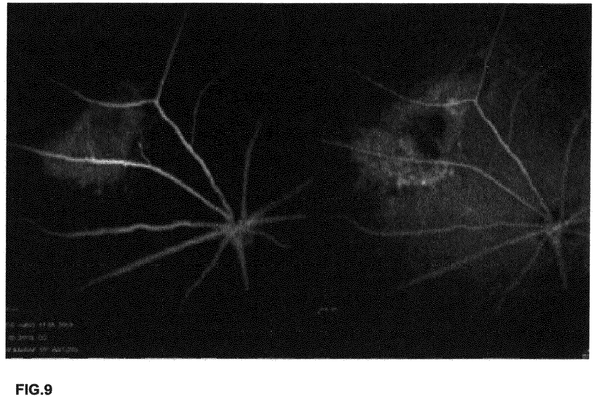

All 60 eyes overexpressing VEGF showed typical CNV lesion-like hyper-fluorescence in FA and ICG imaging (Fig.9) meaning that the VEGF transduction efficacy was 100%.

In the following, eyes successfully transduced with VEGF vector and showing CNV-like lesions will be termed "CNV eyes", the CNV-like lesions "CNV lesion".

All eyes were investigated by angiography. Most CNV lesions showed a typical ring-shaped pattern in both the FA and ICG angiographies. A central hypo-fluorescent area was surrounded by a bright hyper-fluorescent ring especially in FA images (see, Fig. 9 left panel). This pattern correlated well with the OCT analyses showing pronounced subretinal lesions in the hyper-reflective areas. In contrast, the ICG signal showed a rather spotty pattern that usually stretched over a larger area around the hypo-fluorescent center of the lesion.

ICG is a dye that has a very long half-life and binds to luminal proteins. Therefore, it can be recorded at several time points after single intravenous injection if it is retained within the tissue. This occurs, e.g. with protein leakage from CNV vessels into the surrounding tissue.

As shown in Fig. 9 (see, right panel, green channel) and in contrast to the FA signal, the ICG hyper-fluorescence shows a rather spotty pattern around the CNV lesion that spreads over time (within 20 minutes, but also at reinvestigation of ICG without additional dye injections at later time points, here one week after the first angiography session). Finally, this leads to formation of larger fields of single hyper-fluorescent highlights that can cover the whole background of the eye at late time points. These patterns, however, do not dramatically change directly after injection of additional ICG dye.

### Reduction of the thickness of the CNV lesion area by PEDF treatment

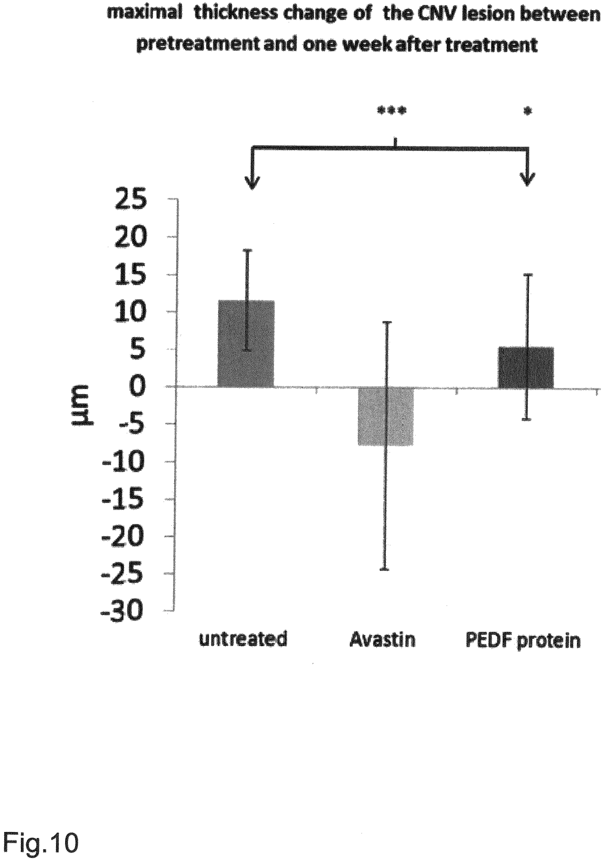

For each eye the area of the whole CNV lesion area (detected by SLO angiography) was screened by OCT. The area of maximal thickness of the lesion was determined and imaged. In these images the maximal thickness was measured. To analyze the changes caused by the treatment with the different agents the differences of the measurement values for each eye for the analysis seven weeks after the subretinal injection of the vector (one week after treatment) and the corresponding values for the six weeks analysis (before treatment) were determined. PEDF inhibited cellular proliferation and fibrosis and therefore reduced the thickness of the CNV significantly compared to the untreated group, but the blood vessels did not collapse completely as in the Avastin group. Thus, the CNVs became flatter in the Avastin group (see, Fig. 10).

### Effects of PEDF on new formation of a healthy choriocapillaris, Bruch's membrane and junctional complexes

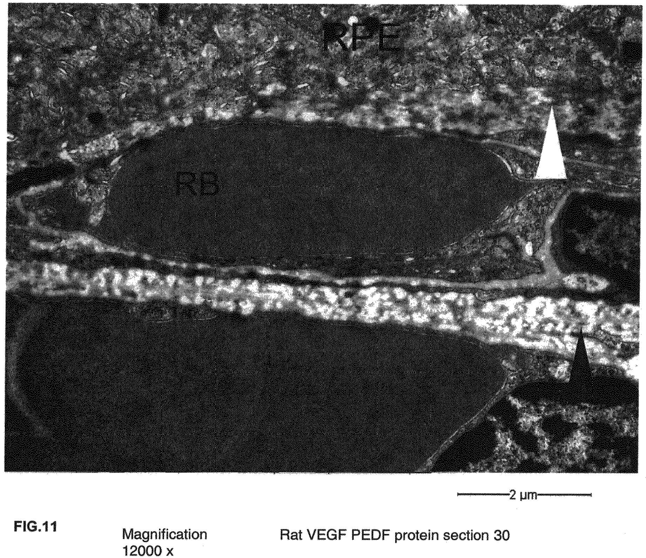

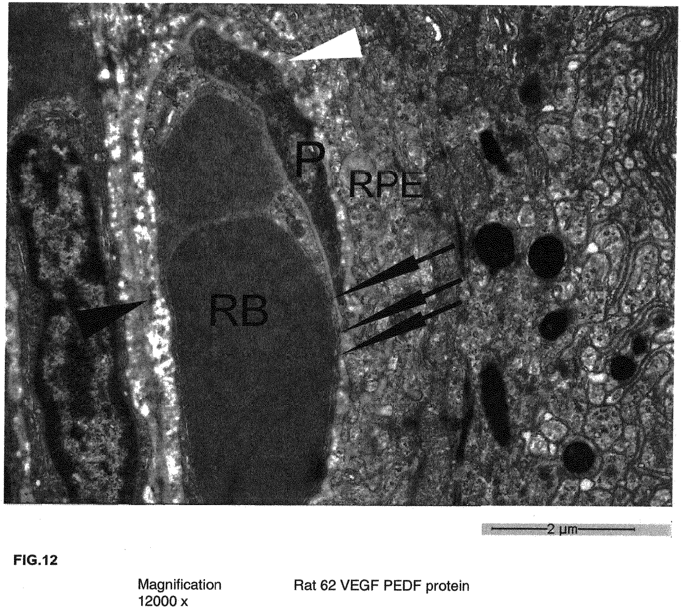

Eyes after PEDF protein treatment were investigated by electron microscopy and eyes after VEGF vector injection without PEDF treatment were used as controls. The most prominent effects of PEDF treatment were that the newly formed choriocapillaris was very similar to the healthy choriocapillaris without any treatment. The newly formed vessels were directly located below the RPE and formed a new Bruch's membrane (see, Fig. 11). The endothelial cells were thin as in healthy vessels, were associated with pericytes, developed fenestrations (see, Fig.12) and did not grow into the subretinal space.

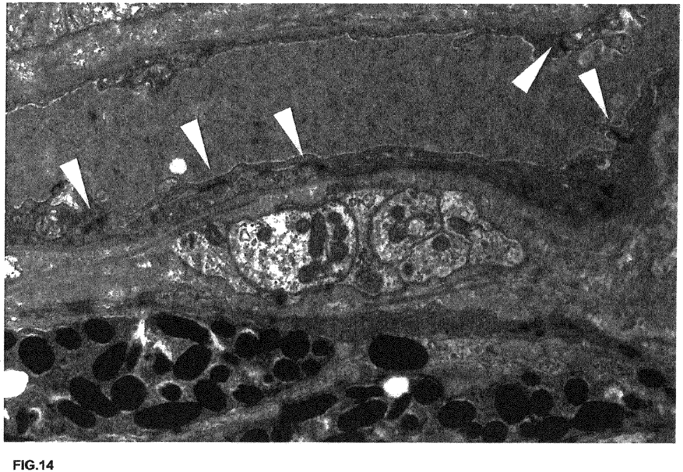

In addition, junctional complexes between retinal pigment epithelial cells (Fig.14) and the endothelial cells of the choriocapillaris (Fig.15) were dramatically enlarged and electron dense compared to only VEGF vector treated eyes. These complexes consist of adherent junctions and tight junctions. Tight junctions also appeared between endothelial cells of the choriocapillaris although they have not been reported in these vessels before. It is generally accepted that the blood retina barrier is built up by the tight junctions of the retinal vessels and the tight junctions of the retinal pigment epithelium. The effect on the junctions is mediated by PEDF in combination with VEGF over expression.

PEDF also reduced dividing of RPE cells and inhibited the formation of intravascular protrusions containing extracellular matrix (see, Fig. 2 and 15). This phenomenon was also described in a rabbit model of CNV (Julien, Kreppel et al. 2008). Such protrusions were not seen after PEDF treatment, which caused formation of monolayered basement membranes in newly formed vessel whereas without treatment the basement membranes were multilayered. Also, the breakthrough of newly formed blood vessels into the subretinal space and retina did not occur after PEDF treatment but were seen without PEDF injection.

### Example 7: Effect of a combination of PEDF and anti-VEGF drug

A combination of PEDF and anti VEGF drugs, for example, Avastin acts synergistically and is supporting the coordinated growth of new functional vessels and also improves the formation of fenestrations in the newly formed choriocapillaris.

### Example 8: PEDF reduces formation of extracelluar matrix in CNV

PEDF reduces formation of extracelluar matrix in CNV. Therefore, scarring which is typical for CNV is minimized and therfore the distance for supply of oxygen and nutrition from the newly formed vessels towards the PRE and photoreceptors is shortened.

### Example 9: Mimicking human AMD by subretinal or intravitral injection of VEGF

Hundred ng VEGF protein (hVEGF Sigma) in 2 µl PBS were injected subretinally or intravitreally into eyes of Long Evans rats. For controls, only PBS was injected.

The eyes were investigated after 1 and 24 hours by electron microscopy and immunocytchemistry. The choriocapillaris changed into labyrinth capillaries as shown in Figs. 2 to 4 and an earlier publication in human CNV's (Schraermeyer, Julien et al. 2015). In addition, there was a prominent augmentation of the extracellular matrix within Bruch's membrane and around the choricapillaris. The protrusion of extracellular matrix which induced the endothelial invaginations into the vessel lumen as shown in Fig. 15 was also present. The synthesis of basement membranes of RPE and vessels was enhanced to multilayers. In addition, the RPE was highly activated and migrated out of the monolayer. Within the choriocapillaris, thrombocytes were activated red blood cells were lysed probably by complement activation and also developed stasis. All these findings were surprisingly already observed 1 - 24 hours after injection, lacked in the control group and mimicked the findings seen in human eyes suffering from AMD.

### References

The complete bibliographic data of the documents recited herein the disclosure of which is incorporated by reference is, if not indicated to the contrary, as follows.
Biesemeier, A., T. Taubitz, S. Julien, E. Yoeruek and U. Schraermeyer (2014). "Choriocapillaris breakdown precedes retinal degeneration in age-related macular degeneration." Neurobiol Aging 35(11): 2562-2573.
Biesemeier, A. K., S. Julien and U. Schraermeyer (2014). "Choroidal neovascularization can help photoreceptors to survive in late AMD." Investigative Ophthalmology & Visual Science 55(13).
Browning, D. J., P. K. Kaiser, P. J. Rosenfeld and M. W. Stewart (2012). "Aflibercept for age-related macular degeneration: a game-changer or quiet addition?" Am J Ophthalmol 154(2): 222-226.
Daha, N. A., N. K. Banda, A. Roos, F. J. Beurskens, J. M. Bakker, M. R. Daha and L. A. Trouw (2011). "Complement activation by (auto-) antibodies." Mol Immunol 48(14): 1656-1665.
Ferrara, N., L. Damico, N. Shams, H. Lowman and R. Kim (2006). "Development of ranibizumab, an anti-vascular endothelial growth factor antigen binding fragment, as therapy for neovascular age-related macular degeneration." Retina 26(8): 859-870.
Fischer, M. D., G. Huber, S. C. Beck, N. Tanimoto, R. Muehlfriedel, E. Fahl, C. Grimm, A. Wenzel, C. E. Reme, S. A. van de Pavert, J. Wijnholds, M. Pacal, R. Bremner and M. W. Seeliger (2009). "Noninvasive, in vivo assessment of mouse retinal structure using optical coherence tomography." PLoS One 4(10): e7507.
Hanhart, J., D. S. Comaneshter, Y. Freier Dror and S. Vinker (2017). "Mortality in patients treated with intravitreal bevacizumab for age-related macular degeneration." BMC Ophthalmol 17(1): 189.
Hanhart, J., D. S. Comaneshter, Y. Freier-Dror and S. Vinker (2018). "Mortality associated with bevacizumab intravitreal injections in age-related macular degeneration patients after acute myocardial infarct: a retrospective population-based survival analysis." Graefes Arch Clin Exp Ophthalmol 256(4): 651-663.
Hanhart, J., D. S. Comaneshter and S. Vinker (2018). "Mortality after a cerebrovascular event in age-related macular degeneration patients treated with bevacizumab ocular injections." Acta Ophthalmol 96(6): e732-e739.
He, T., J. Hu, G. Yan, L. Li, D. Zhang, Q. Zhang, B. Chen and Y. Huang (2015). "Pigment epithelium-derived factor regulates microvascular permeability through adipose triglyceride lipase in sepsis." Clin Sci (Lond) 129(1): 49-61.
Huber, G., S. C. Beck, C. Grimm, A. Sahaboglu-Tekgoz, F. Paquet-Durand, A. Wenzel, P. Humphries, T. M. Redmond, M. W. Seeliger and M. D. Fischer (2009). "Spectral domain optical coherence tomography in mouse models of retinal degeneration." Invest Ophthalmol Vis Sci 50(12): 5888-5895.
Julien, S., A. Biesemeier and U. Schraermeyer (2013). "In vitro induction of protein complexes between bevacizumab, VEGF-A(1)(6)(5) and heparin: explanation for deposits observed on endothelial veins in monkey eyes." Br J Ophthalmol 97(4): 511-517.
Julien, S., A. Biesemeier, T. Taubitz and U. Schraermeyer (2014). "Different effects of intravitreally injected ranibizumab and aflibercept on retinal and choroidal tissues of monkey eyes." Br J Ophthalmol 98(6): 813-825.
Julien, S., F. Kreppel, S. Beck, P. Heiduschka, V. Brito, S. Schnichels, S. Kochanek and U. Schraermeyer (2008). "A reproducible and quantifiable model of choroidal neovascularization induced by VEGF A165 after subretinal adenoviral gene transfer in the rabbit." Mol Vis 14: 1358-1372.
King, G. L. and K. Suzuma (2000). "Pigment-epithelium-derived factor - A key coordinator of retinal neuronal and vascular functions." New England Journal of Medicine 342(5): 349-351.
Meyer, C. H. and F. G. Holz (2011). "Preclinical aspects of anti-VEGF agents for the treatment of wet AMD: ranibizumab and bevacizumab." Eye (Lond) 25(6): 661-672.
Meyer, T., L. Robles-Carrillo, T. Robson, F. Langer, H. Desai, M. Davila, M. Amaya, J. L. Francis and A. Amirkhosravi (2009). "Bevacizumab immune complexes activate platelets and induce thrombosis in FCGR2A transgenic mice." J Thromb Haemost 7(1): 171-181.
Papadopoulos, N., J. Martin, Q. Ruan, A. Rafique, M. P. Rosconi, E. Shi, E. A. Pyles, G. D. Yancopoulos, N. Stahl and S. J. Wiegand (2012). "Binding and neutralization of vascular endothelial growth factor (VEGF) and related ligands by VEGF Trap, ranibizumab and bevacizumab." Angiogenesis 15(2): 171-185.
Peters, S., P. Heiduschka, S. Julien, F. Ziemssen, H. Fietz, K. U. Bartz-Schmidt, G. Tubingen Bevacizumab Study and U. Schraermeyer (2007). "Ultrastructural findings in the primate eye after intravitreal injection of bevacizumab." Am J Ophthalmol 143(6): 995-1002.
Rolling, F., G. Le Meur, K. Stieger, A. J. Smith, M. Weber, J. Y. Deschamps, D. Nivard, A. Mendes-Madeira, N. Provost, Y. Pereon, Y. Cherel, R. R. Ali, C. Hamel, P. Moullier and F. Rolling (2006). "Gene therapeutic prospects in early onset of severe retinal dystrophy: restoration of vision in RPE65 Briard dogs using an AAV serotype 4 vector that specifically targets the retinal pigmented epithelium." Bull Mem Acad R Med Belg 161(10-12): 497-508; discussion 508-499.
Schraermeyer, U. and S. Julien (2012). "Formation of immune complexes and thrombotic microangiopathy after intravitreal injection of bevacizumab in the primate eye." Graefes Arch Clin Exp Ophthalmol 250(9): 1303-1313.
Schraermeyer, U. and S. Julien (2013). "Effects of bevacizumab in retina and choroid after intravitreal injection into monkey eyes." Expert Opin Biol Ther 13(2): 157-167.
Schraermeyer, U., S. Julien, A. Biesemeier, K. U. Bartz-Schmidt and H. Wolburg (2015). "A new kind of labyrinth-like capillary is responsible for leakage from human choroidal neovascular endothelium, as investigated by high-resolution electron microscopy." Graefes Arch Clin Exp Ophthalmol 253(5): 681-689.
Schutze, C., M. Wedl, B. Baumann, M. Pircher, C. K. Hitzenberger and U. Schmidt-Erfurth (2015). "Progression of retinal pigment epithelial atrophy in antiangiogenic therapy of neovascular age-related macular degeneration." Am J Ophthalmol 159(6): 1100-1114 e1101. Treister, A. D., P. L. Nesper, A. E. Fayed, M. K. Gill, R. G. Mirza and A. A. Fawzi (2018). "Prevalence of Subclinical CNV and Choriocapillaris Nonperfusion in Fellow Eyes of Unilateral Exudative AMD on OCT Angiography." Transl Vis Sci Technol 7(5): 19.
Spaide RF, Klancnik JM, Cooney MJ. Retinal Vascular Layers Imaged by Fluorescein Angiography and Optical Coherence Tomography Angiography. JAMA Ophthalmol. 2015;133(1):45.
Wang, F., K. G. Rendahl, W. C. Manning, D. Quiroz, M. Coyne and S. S. Miller (2003). "AAV-mediated expression of vascular endothelial growth factor induces choroidal neovascularization in rat." Invest Ophthalmol Vis Sci 44(2): 781-790.

The features of the present invention disclosed in the specification, the claims, the sequence listing and/or the drawings may both separately and in any combination thereof be material for realizing the invention in various forms thereof.

## Claims

1. A pigment epithelium-derived factor (PEDF) for use in a method for treatment and/or prevention of a disease, wherein the method comprises administering PEDF to a subject and wherein treatment and/or prevention of a disease comprises inhibiting labyrinth capillary formation, inducing growth of choriocapillaris, tightening choriocapillaris, inhibiting extracellular matrix formation, protecting choriocapillaris, and/or guiding vessel development.

2. The pigment epithelium-derived factor (PEDF) for use of claim 1, wherein the disease is an eye disease.

3. The pigment epithelium-derived factor (PEDF) for use of claim 2, wherein the eye disease is macular degeneration, preferably macular degeneration is age-related macular degeneration (AMD), more preferably dry age-related macular degeneration or wet age-related macular degeneration.

4. The pigment epithelium-derived factor (PEDF) for use of claim 2, wherein the disease is selected from the group comprising central serous chorioretinopathy, diabetic retinopathy, rubeosis iridis, corneal neovascularization, polypoidal choroidal vasculopathy and retinopathy of the prematurity.

5. The pigment epithelium-derived factor (PEDF) for use of any one of claims 2 to 4, wherein labyrinth capillary formation is labyrinth capillary formation in an eye, preferably in eye disease.

6. The pigment epithelium-derived factor (PEDF) for use of any one of claims 1 to 5, wherein inducing growth of choriocapillaris comprises or is inducing growth of new choriocapillaris.

7. The pigment epithelium-derived factor (PEDF) for use of any one of claims 1 to 6, wherein inducing growth of choriocapillaris provides choriocapillaris which are capable of replacing original choriocapillaris, preferably original choriocapillaris are diseased choriocapillaris.

8. The pigment epithelium-derived factor (PEDF) for use of any one of claims 1 to 7, wherein tightening choriocapillaris comprises tightening pathological choriocapillaris.

9. The pigment epithelium-derived factor (PEDF) for use of any one of claims 1 to 8, wherein inhibiting extracellular matrix formation comprises inhibition of extracellular matrix formation towards the lumen of a blood vessel and/or around a blood vessel.

10. The pigment epithelium-derived factor (PEDF) for use of any one of claims 1 to 9, wherein protecting choriocapillaris comprises protecting choriocapillaris from the damaging effect of an anti-VEGF drug.

11. The pigment epithelium-derived factor (PEDF) for use of claim 12, wherein protecting choriocapillaris comprises protecting choriocapillaris from the damaging effect of withdrawal of an anti-VEGF drug.

12. The pigment epithelium-derived factor (PEDF) for use of any one of claim 1 to 11, wherein guiding vessel development comprises development of a functional blood vessel, preferably a functional blood vessel from a pathological blood vessel.

13. The pigment epithelium-derived factor (PEDF) for use of claim 12, wherein the pathological blood vessel is the result of a pathological condition.

14. The pigment epithelium-derived factor (PEDF) for use of any one of claim 1 to 13, wherein PEDF is administered intravitreally or sub-retinally.

15. The pigment epithelium-derived factor (PEDF) for use of any one of claim 1 to 14, wherein the method further comprises applying an anti-VEGF therapy, preferably the anti-VEGF therapy comprises administration to the subject of an anti-VEGF drug, wherein the anti-VEGF drug is selected from the group comprising pegaptanib, ranibizumab, bevacizumab and aflibercept.

16. A method for the screening of a pigment epithelium-derived factor (PEDF) analog, wherein the method comprises
- intravitreally or subretinally administering VEGF into an animal model,
- administering a pigment epithelium-derived factor (PEDF) analog candidate into the animal model,
- determining the effect of the pigment epithelium-derived factor (PEDF) analog candidate after 1 to 72 h,
wherein the pigment epithelium-derived factor (PEDF) analog candidate is a pigment epithelium-derived factor (PEDF) analog if the effect of VEGF is blocked, no leakage of the blood vessels occurs, no increase in the extracellular matrix occurs, and/or no thickening of the Bruch's membrane occurs.

17. A method for the screening of an anti-VEGF agent, wherein the method comprises
- intravitreally or subretinally administering VEGF into an animal model,
- administering an anti-VEGF agent candidate into the animal model
- determining the effect of the anti-VEGF agent candidate after 1 to 72 h,
wherein the anti-VEGF agent candidate is an anti-VEGF agent if the effect of VEGF is blocked, no leakage of the blood vessels occurs, no increase in the extracellular matrix occurs and/or no thickening of the Bruch's membrane occurs.


SEQUENCE LISTING

<110>  Curebiotec GmbH

<120>  Method for the treatment of a disease using pigment
       epithelium-derived factor (PEDF)

<130>  S 10122 EP

<160>  2

<170>  PatentIn version 3.5

<210>  1
<211>  399
<212>  PRT
<213>  Homo sapiens

<220>
<221>  MISC_FEATURE
<223>  Pigment epithelium-derived factor (PEDF)

<400>  1

Gln Asn Pro Ala Ser Pro Pro Glu Glu Gly Ser Pro Asp Pro Asp Ser
1               5                   10                  15

Thr Gly Ala Leu Val Glu Glu Glu Asp Pro Phe Phe Lys Val Pro Val
            20                  25                  30

Asn Lys Leu Ala Ala Ala Val Ser Asn Phe Gly Tyr Asp Leu Tyr Arg
        35                  40                  45

Val Arg Ser Ser Thr Ser Pro Thr Thr Asn Val Leu Leu Ser Pro Leu
    50                  55                  60

Ser Val Ala Thr Ala Leu Ser Ala Leu Ser Leu Gly Ala Glu Gln Arg
65                  70                  75                  80

Thr Glu Ser Ile Ile His Arg Ala Leu Tyr Tyr Asp Leu Ile Ser Ser
                85                  90                  95

Pro Asp Ile His Gly Thr Tyr Lys Glu Leu Leu Asp Thr Val Thr Ala
            100                 105                 110

Pro Gln Lys Asn Leu Lys Ser Ala Ser Arg Ile Val Phe Glu Lys Lys
        115                 120                 125

Leu Arg Ile Lys Ser Ser Phe Val Ala Pro Leu Glu Lys Ser Tyr Gly
    130                 135                 140

Thr Arg Pro Arg Val Leu Thr Gly Asn Pro Arg Leu Asp Leu Gln Glu
145                 150                 155                 160

Ile Asn Asn Trp Val Gln Ala Gln Met Lys Gly Lys Leu Ala Arg Ser
                165                 170                 175

Thr Lys Glu Ile Pro Asp Glu Ile Ser Ile Leu Leu Leu Gly Val Ala
            180                 185                 190

His Phe Lys Gly Gln Trp Val Thr Lys Phe Asp Ser Arg Lys Thr Ser
        195                 200                 205

Leu Glu Asp Phe Tyr Leu Asp Glu Glu Arg Thr Val Arg Val Pro Met
    210                 215                 220

Met Ser Asp Pro Lys Ala Val Leu Arg Tyr Gly Leu Asp Ser Asp Leu
225                 230                 235                 240

Ser Cys Lys Ile Ala Gln Leu Pro Leu Thr Gly Ser Met Ser Ile Ile
                245                 250                 255

Phe Phe Leu Pro Leu Lys Val Thr Gln Asn Leu Thr Leu Ile Glu Glu
            260                 265                 270

Ser Leu Thr Ser Glu Phe Ile His Asp Ile Asp Arg Glu Leu Lys Thr
        275                 280                 285

Val Gln Ala Val Leu Thr Val Pro Lys Leu Lys Leu Ser Tyr Glu Gly
    290                 295                 300

Glu Val Thr Lys Ser Leu Gln Glu Met Lys Leu Gln Ser Leu Phe Asp
305                 310                 315                 320

Ser Pro Asp Phe Ser Lys Ile Thr Gly Lys Pro Ile Lys Leu Thr Gln
                325                 330                 335

Val Glu His Arg Ala Gly Phe Glu Trp Asn Glu Asp Gly Ala Gly Thr
            340                 345                 350

Thr Pro Ser Pro Gly Leu Gln Pro Ala His Leu Thr Phe Pro Leu Asp
        355                 360                 365

Tyr His Leu Asn Gln Pro Phe Ile Phe Val Leu Arg Asp Thr Asp Thr
    370                 375                 380

Gly Ala Leu Leu Phe Ile Gly Lys Ile Leu Asp Pro Arg Gly Pro
385                 390                 395

<210>  2

<211>  1000
<212>  DNA
<213>  Homo sapiens

<220>
<221>  misc_feature
<223>  Coding sequence of pigment epithelium-derived factor (PEDF)

<400>  2
ggacgctgga ttagaaggca gcaaaaaaag atctgtgctg gctggagccc cctcagtgtg      60

caggcttaga gggactaggc tgggtgtgga gctgcagcgt atccacaggc cccaggatgc      120

aggccctggt gctactcctc tgcattggag ccctcctcgg gcacagcagc tgccagaacc      180

ctgccagccc cccggaggag ggctccccag accccgacag cacaggggcg ctggtggagg      240

aggaggatcc tttcttcaaa gtccccgtga acaagctggc agcggctgtc tccaacttcg      300

gctatgacct gtaccgggtg cgatccagca tgagccccac gaccaacgtg ctcctgtctc      360

ctctcagtgt ggccacggcc ctctcggccc tctcgctggg agcggacgag cgaacagaat      420

ccatcattca ccgggctctc tactatgact tgatcagcag cccagacatc catggtacct      480

ataaggagct ccttgacacg gtcactgccc cccagaagaa cctcaagagt gcctcccgga      540

tcgtctttga gaagaagctr cgcataaaat ccagctttgt ggcacctctg gaaaagtcat      600

atgggaccag gcccagagtc ctgacgggca accctcgctt ggacctgcaa gagatcaaca      660

actgggtgca ggcgcagatg aaagggaagc tcgccaggtc cacaaaggaa attcccgatg      720

agatcagcat tctccttctc ggtgtggcgc acttcaaggg gcagtgggta acaaagtttg      780

actccagaaa gacttccctc gaggatttct acttggatga agagaggacc gtgagggtcc      840

ccatgatgtc ggaccctaag gctgttttac gctatggctt ggattcagat ctcagctgca      900

agattgccca gctgcccttg accggaagca tgagtatcat cttcttcctg cccctgaaag      960

tgacccagaa tttgaccttg atagaggaga gcctcacctc      1000